# EUROPÄISCHE PATENTSCHRIFT

(11) **EP 3 363 346 B1**
(45) Veröffentlichungstag und Bekanntmachung des Hinweises auf die Patenterteilung: **20.05.2020**
(21) Anmeldenummer: 18151589.1
(22) Anmeldetag: 15.01.2018
(51) Int. Cl.: A61B 3/10, G02C 13/00, A61B 3/14

(54) **COMPUTERIMPLEMENTIERTES VERFAHREN ZUR DETEKTION EINES HORNHAUTSCHEITELS**
COMPUTER-IMPLEMENTED METHOD FOR DETECTING A CORNEA VERTEX
PROCÉDÉ MIS EN UVRE PAR ORDINATEUR DESTINÉ À LA DÉTECTION D'UN SOMMET DE LA CORNÉE

(30) Priorität: 27.01.2017 EP 17153558
(43) Veröffentlichungstag der Anmeldung: 22.08.2018
(60) Teilanmeldung: 19190040.6
(73) Patentinhaber: Carl Zeiss Vision International GmbH, 73430 Aalen (DE); Carl Zeiss AG, 73447 Oberkochen (DE)
(72) Erfinder: NIEUWENHUIS, Claudia, 73457 Essingen (DE); SCHWARZ, Oliver, 73479 Ellwangen (DE); GAMPERLING, Michael, 89340 Leipheim (DE)
(74) Vertreter: Patentanwälte Bregenzer und Reule Partnerschaftsgesellschaft mbB

(56) Entgegenhaltungen:
- JP-A- 2007 206 211
- JP-B2- 5 854 861
- US-A- 5 573 006
- US-A1- 2003 081 173
- US-A1- 2003 123 026
- US-A1- 2011 007 269
- US-A1- 2012 148 159
- US-A1- 2016 379 093

## Beschreibung

Die Erfindung betrifft ein computerimplementiertes Verfahren zur Detektion eines Hornhautscheitels gemäß Oberbegriff des Anspruchs 1.

Die Hornhautscheitel eines Probanden sind wichtige Punkte bei der Bestimmung von Zentrierparametern für die Brillenanpassung. Zentrierparameter werden benutzt, um Brillengläser korrekt in einer Brillenfassung anzuordnen bzw. zu zentrieren, so dass die Brillengläser in korrekter Position relativ zu den Augen der die Brille tragenden Person angeordnet sind. Dabei handelt es sich zum Teil um anatomische Parameter der betreffenden Person, wie beispielsweise den Pupillenabstand, zum Teil um rein fassungsspezifische Parameter wie die Fassungsscheibenbreite oder die Fassungsscheibenhöhe und zum Teil um Kombinationen aus anatomischen und fassungsspezifischen Parametern, wie beispielsweise den Hornhautscheitelabstand und die Durchblickshöhe. Einen Überblick über die gängigen Zentrierparameter gibt die DIN EN ISO 13666 vom Oktober 2013.

Eine zumindest näherungsweise Bestimmung der Position des Hornhautscheitels im Raum und vorzugsweise der Position beider Hornhautscheitel im Raum kann bei einer weiteren Auswertung vorteilhaft für die Bestimmung von Zentrierparametern, insbesondere für die Bestimmung des

Hornhautscheitelabstands herangezogen werden. Bisher wurden die Hornhautscheitelpositionen manuell in den Front- und Seitenbildern annotiert.

Dabei wurde je nach Vorliebe des Optikers eine reflexbasierte oder pupillenbasierte Annotation favorisiert. Bei der reflexbasierten Annotation wird mit Hilfe einer LED ein Licht-Reflex auf der Pupille erzeugt, der leichter zu detektieren ist als die Pupillenmitte. Viele Optiker bevorzugen aber nach wie vor die pupillenbasierte Annotation.

Ein Verfahren der eingangs genannten Art ist aus der US 2011/0007269 A1 bekannt. Bei diesem Verfahren werden, ebenso wie bei den aus der US 2003/0123026 A1, der JP 2007/206211 A und der US 2007/195266 A1 bekannten Verfahren, Stereoalgorithmen benutzt, die darauf beruhen, Korrespondenzen in Bildern zu finden. Voraussetzung ist jeweils ein großer Überlapp der Bilder, so dass die Wahl der Kameraanordnungen stark eingeschränkt ist. Die Kameras mit stark unterschiedlichen Aufnahmerichtungen im Raum anzuordnen ist dabei nicht möglich.

Es ist Aufgabe der Erfindung, ein Verfahren bereitzustellen, mit dem die Position eines Hornhautscheitels im Raum einfacher detektiert werden kann.

Ausgehend von der US 2011/0007269 A1 löst das Verfahren gemäß Anspruch 1 die Aufgabe, das vorbekannte Verfahren durch eine Vorverarbeitung von Daten zu vereinfachen.

Mit dem erfindungsgemäßen Verfahren wird eine Voraussetzung dafür geschaffen, Zentrierparameter wie die Pupillendistanz und den Hornhautscheitelabstand zu berechnen, und zwar sowohl bei 'realer' Zentrierung mit einer realen Brillenfassung, als auch bei virtueller Zentrierung, wobei dem Bild eines Probanden virtuell eine Brille oder Brillenfassung "aufgesetzt" wird, wenn deren geometrische Daten vorliegen. Dabei werden vorzugsweise gleichzeitig aufgenommene kalibrierte Bilder bereitgestellt. Deren Kalibrierung umfasst die extrinsischen Eigenschaften der die Bilder aufnehmenden Kameras oder der die Bilder nacheinander aufnehmenden Kamera wie die relative Ausrichtung ihrer optischen Achsen sowie die relative Anordnung zueinander im Raum, sowie ihre intrinsischen Eigenschaften, also die Eigenschaften der Kameras selbst, die definieren, wie ein Punkt im Raum, der sich im internen Koordinatensystem der jeweiligen Kamera befindet, auf die Koordinaten der Pixel des aufgenommenen Bildes abgebildet wird. Eine ausführliche Beschreibung der Kalibrierung von Kameras findet sich im Lehrbuch "Multiple View Geometry in Computer Vision" von Richard Hartley und Andrew Zisserman, 2. Auflage, Cambridge University Press 2004, und dort insbesondere auf Seite 8. Dabei wird bevorzugt, dass bei der geometrischen Positionsbestimmung des Hornhautscheitels im Raum ein Triangulationsverfahren verwendet wird.

Die in einer ersten Näherung bestimmte Position des Hornhautscheitels im Raum kann einer Korrekturrechnung unterzogen werden. Die Art der Korrekturrechnung ist dann abhängig von der Art, wie die Position des Hornhautscheitels im Raum in der ersten Näherung bestimmt wird.

Gemäß einer ersten Ausführungsform der Erfindung ist vorgesehen, bei einer sogenannten pupillenbasierten Auswertung die Position des Hornhautscheitels im Raum in erster Näherung als Schnittpunkt eines die Hornhaut tangierenden Sichtstrahls einer das Seitenbild aufnehmenden Seitenkamera mit einem auf die Pupille gerichteten Sichtstrahl einer das Frontbild aufnehmenden Frontkamera zu bestimmen. Ist dies der Fall, wird vorteilhaft mittels der Korrekturrechnung die Position des Hornhautscheitels gemäß a = q + µ ^{∗} v + µ ^{∗} w berechnet, wobei a der Positionsvektor des Hornhautscheitels im Raum nach Durchführung der Korrekturrechnung, q die Position des Hornhautscheitels in erster Näherung, µ ein Erfahrungswert oder ein tatsächlicher Wert für den Abstand zwischen dem Pupillenzentrum und dem Hornhautscheitel, v ein Einheitsvektor der Raumrichtung vom Pupillenzentrum zur Frontkamera und w ein Einheitsvektor der durch das Zentrum der Hornhautkugel verlaufenden Blickrichtung ist.

Gemäß eines nicht zur Erfindung gehörenden Verfahrens ist vorgesehen, eine reflexbasierte Auswertung vorzunehmen, indem bei der Aufnahme der Bilder ein Lichtblitz vorzugsweise mittels einer LED erzeugt wird, wobei die Position des Hornhautscheitels im Raum in erster Näherung als die Position des Reflexpunkts des Lichtblitzes auf der Hornhaut bestimmt wird. Ausgehend von dieser ersten Näherung wird, wenn der Lichtblitz mittels einer mittig vor dem Gesicht des Probanden angeordneten Lichtquelle erzeugt wird, mittels der Korrekturrechnung die Position des Hornhautscheitels in horizontaler Richtung ausgehend vom Reflexpunkt mittels Addition von Δx =+/ -r ^{∗} sin (½ ^{∗} (arccos z/a + arctan x/(z-v))) zur x-Koordinate näherungsweise korrigiert, wobei r ein Erfahrungswert oder ein tatsächlicher Wert für den Hornhautradius, a der Abstand des optischen Zentrums einer das Frontbild aufnehmenden Frontkamera zum Reflexpunkt, x und z die x- und z-Koordinaten des Reflexpunkts in einem Koordinatensystem mit dem Nullpunkt im optischen Zentrum der Frontkamera, wobei die Blickrichtung der Frontkamera der z-Richtung entspricht und die x-Richtung horizontal orthogonal zur z-Richtung steht und in z-Richtung betrachtet nach rechts weist, und v der Abstand der den Lichtblitz erzeugenden Lichtquelle vom optischen Zentrum der Frontkamera in z-Richtung ist. Ergänzend oder alternativ hierzu wird mittels der Korrekturrechnung die Position des Hornhautscheitels in y-Richtung ausgehend vom Reflexpunkt mittels Addition von Δy =+/ -r ^{∗} sin ½ ^{∗} (arctan l/(d-v)) berechnet, wobei r ein Erfahrungswert oder ein tatsächlicher Wert für den Hornhautradius, d der Abstand des optischen Zentrums der Frontkamera zum Reflexpunkt, v der Abstand der den Lichtblitz erzeugenden Lichtquelle vom optischen Zentrum der Frontkamera in z-Richtung und I der Abstand der Lichtquelle vom optischen Zentrum der Frontkamera in y-Richtung ist, wobei die y-Richtung orthogonal zur x-Richtung und zur z-Richtung ist und im Raum nach oben weist.

Unter optischem Zentrum versteht man das Projektionszentrum der Kamera.

Dabei ist das Pluszeichen in x-Richtung anzuwenden, wenn der Hornhautscheitel des aus der Sicht des Probanden linken Auges detektiert wird, das Minuszeichen ist für das aus der Sicht des Probanden rechte Auge anzuwenden. In y-Richtung ist das Pluszeichen anzuwenden, wenn die den Lichtblitz aussendende Lichtquelle auf einer niedrigeren Höhe positioniert ist als die Frontkamera, das Minuszeichen ist anzuwenden, wenn sie in einer größeren Höhe montiert ist.

Die beiden vorstehend angegebenen Korrekturformeln ergeben sich aus folgenden Überlegungen:
Um die 3D-Koordinaten eines Punktes zu bestimmen, muss man den gleichen Punkt in mindestens zwei Ansichten detektieren, d.h. hier in Front- und einem Seitenbild. Da es sich beim Glaskörper des Auges um eine Art Kugel handelt, ist es nicht ausreichend, den Reflex in zwei Ansichten zu detektieren. Zum einen ist der Reflex im Seitenbild kaum oder gar nicht sichtbar durch den großen Aufnahmewinkel, zum anderen wandert der Reflex auf der Kugel je nach Blickrichtung der Kamera, was zu sehr ungenauen 3D-Punkten führen würde.

Auch ohne Reflex ist eine Korrektur der 3D-Position notwendig, da die Pupille leicht hinter der Glaskörperoberfläche liegt und die Kamera-Sichtstrahlen sich durch die Kugelform leicht vor der Kugel schneiden.

Erfindungsgemäß wird die Pupille bzw. der Reflexpunkt mittels Merkmalsextraktion und/oder Merkmalsmatchings (Merkmalsvergleich) und/oder mittels maschinellen Lernens durch Vergleich mit einer Vielzahl vorbekannter Daten detektiert. Diesem Verfahrensschritt kann eine Gesichtsdetektion und/oder eine Detektion von Gesichtsmerkmalen wie den Augen als Vorverarbeitung vorausgehen, bei der detektiert wird, welche Bilddaten zum Gesicht des Probanden gehören, so dass nur diese Daten in die Detektion einfließen. Zur weiteren Vereinfachung des Verfahrens kann vorgesehen sein, dass aus dem Frontbild ein auf den Hornhautscheitel gerichteter Sichtstrahl der Frontkamera berechnet und in das Seitenbild projiziert wird und dass der Hornhautscheitel auf dem ins Seitenbild projizierten Sichtstrahl detektiert wird.

Um die Bestimmung des Hornhautscheitels im Raum für beide Augen vornehmen zu können, wird bevorzugt, dass mindestens ein gleichzeitig mit dem ersten und zweiten Bild (Frontbild und Seitenbild) seitlich bezüglich des Kopfes aufgenommenes, kalibriertes drittes Bild bzw. weiteres Seitenbild bereitgestellt wird.

Das mindestens eine Seitenbild und gegebenenfalls das mindestens eine weitere Seitenbild können grundsätzlich aus Aufnahmerichtungen aufgenommen werden, die mit der Aufnahmerichtung des Frontbilds nur einen kleinen Winkel einschließen. Bei den erfindungsgemäßen Verfahren ist es aber nicht notwendig, dass sich die Front- und Seitenbilder überlappen. Eventuell einander überlappende Bereiche der Bilder werden zweckmäßig zur Bestimmung des Hornhautscheitels nicht herangezogen. Auf diese Weise ist es möglich, dass die Aufnahmerichtung des Frontbilds mit der Aufnahmerichtung des Seitenbilds und gegebenenfalls mit der Aufnahmerichtung des weiteren Seitenbilds einen Winkel von mehr als 60 bis 120 Grad und vorzugsweise von 90 Grad einschließt. Vorzugsweise wird das erfindungsgemäße computerimplementierte Verfahren mit einer Vorrichtung durchgeführt, wie sie grundsätzlich in Anspruch 4 und im Detail in der folgenden Figurenbeschreibung beschrieben wird.

Die erfindungsgemäß bestimmte Position des Hornhautscheitels im Raum kann vorteilhaft zum Zentrieren eines Brillenglases in einer Brillenfassung und/oder zum Einschleifen eines Brillenglases in eine Brillenfassung herangezogen werden. Dabei wird in einem Verfahrensschritt unter Heranziehung der Position des mindestens einen Hornhautscheitels im Raum mindestens ein Zentrierparameter, insbesondere der Hornhautscheitelabstand, oder, bei Vorliegen beider Hornhautscheitel, die Pupillendistanz, bestimmt, und in einem weiteren Verfahrensschritt das mindestens eine Brillenglas mit dem bzw. den im vorigen Verfahrensschritt bestimmten Zentrierparameter(n) in der Brillenfassung zentriert oder es wird das mindestens eine Brillenglas basierend auf dem bzw. den im vorigen Verfahrensschritt bestimmten Zentrierparameter(n) für eine Anordnung in der Brillenfassung eingeschliffen. Auf diese Weise können Brillengläser und Brillen hergestellt werden.

Im Folgenden wird die Erfindung anhand eines in der Zeichnung schematisch dargestellten Ausführungsbeispiels näher erläutert. Es zeigen:

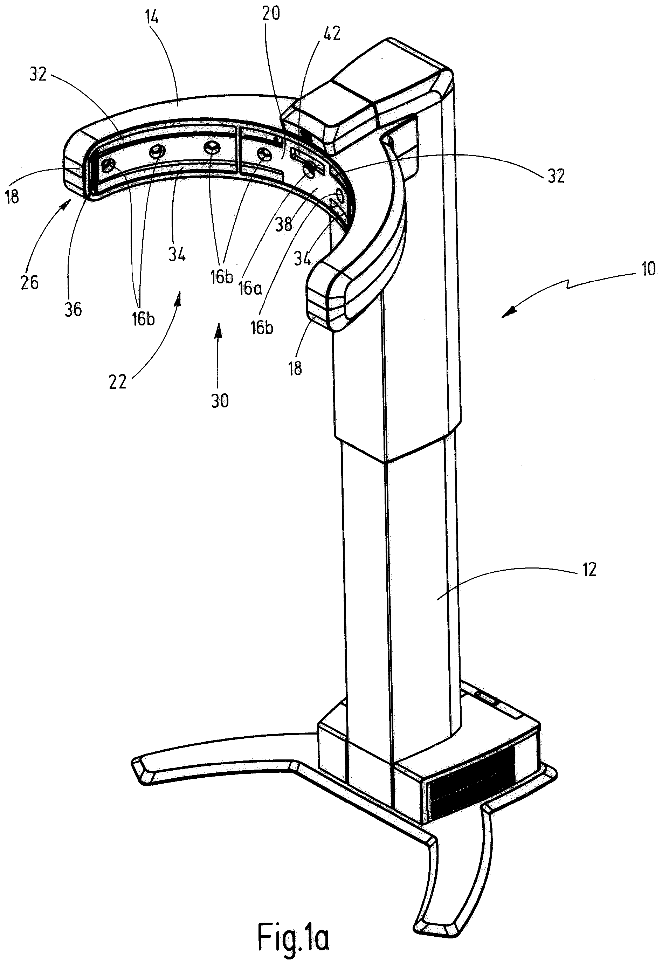

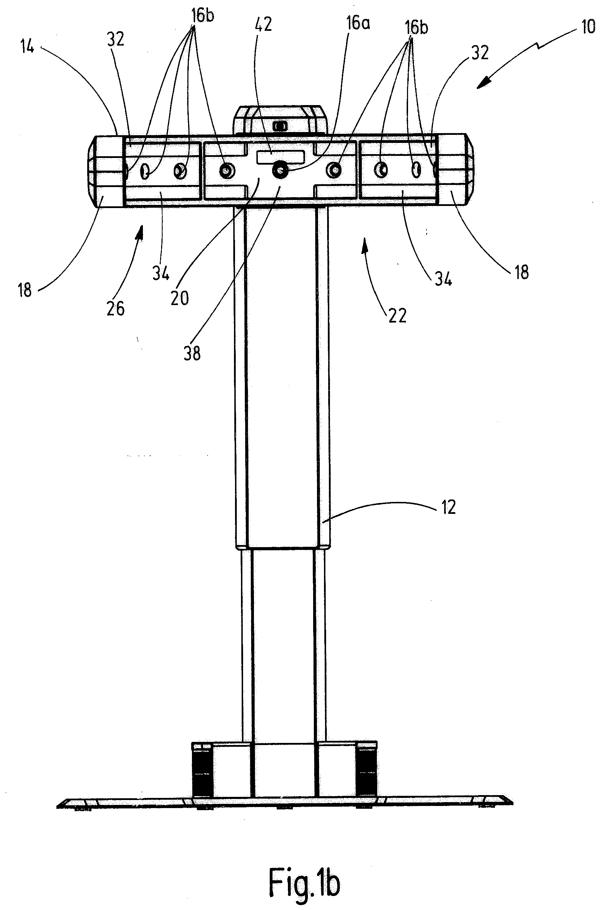

- Figur 1a, b: eine Vorrichtung zur Bestimmung von Zentrierparametern in perspektivischer Ansicht und in einer Ansicht von vorne;
- Figur 2: eine Veranschaulichung der Korrekturrechnung bei pupillenbasierter Bestimmung der Position des Hornhautscheitels und
- Figur 3a,b: eine Veranschaulichung der Korrekturrechnung bei reflexbasierter Bestimmung der Position des Hornhautscheitels.

Die in der Zeichnung dargestellte Vorrichtung 10 dient der Bestimmung von Zentrierparametern für die Brillenanpassung. Sie weist eine Säule 12 auf, die höhenverstellbar einen Kameraträger 14 trägt, welcher wiederum eine Anzahl Kameras 16a, 16b trägt. Der Kameraträger 14 ist in Draufsicht näherungsweise kreisförmig gebogen und erstreckt sich zwischen zwei freien Enden 18, welche im Abstand zueinander angeordnet sind. Nach vorne, also zur Säule 12 hin, und zu den Seiten umschließt eine Innenfläche 20 des Kameraträgers 14 einen Innenraum 22, in dem sich bei der Aufnahme von Bildern durch die Kameras 16a, 16b der Kopf eines Probanden befindet. Die Innenfläche 20 ist in einer Richtung, die zwischen den freien Enden 18 verläuft, konkav gebogen und weist beispielsweise die Form eines Abschnitts einer Zylindermantelfläche auf, wobei der Zylinder eine kreisrunde oder ovale Grundfläche haben kann. Um den Kameraträger 14 bezüglich des Kopfs des Probanden auf der richtigen Höhe positionieren zu können, ist in der Säule 12 eine nicht näher dargestellte Hubeinrichtung angeordnet, mit der der Kameraträger 14 motorisch angetrieben auf und ab bewegt werden kann.

Alle Kameras 16a, 16b sind in einer sich zwischen den freien Enden 18 erstreckenden Kameraanordnung 26 angeordnet. Im vorliegenden Ausführungsbeispiel ist die Kameraanordnung 26 als Kamerareihe 26 ausgebildet, deren Kameras 16a, 16b sich alle in derselben Höhe befinden, wobei ihre optischen Achsen auf den Innenraum 22 gerichtet sind. Im vorliegenden Ausführungsbeispiel umfasst die Kamerareihe 26 eine in der Mitte des Kameraträgers 14 angeordnete Frontkamera 16a, deren optische Achse frontal auf das Gesicht des Probanden gerichtet ist, sowie acht paarweise symmetrisch bezüglich einer durch die optische Achse der Frontkamera 16a verlaufenden senkrechten Symmetrieebene angeordnete Seitenkameras 16b von denen jeweils vier von links und von rechts auf das Gesicht des Probanden gerichtet sind. Die Kameras 16a, 16b sind zudem kalibriert, so dass sie gleichzeitig kalibrierte Bilder des Probanden aufnehmen können. Die Kalibrierung umfasst die extrinsischen Eigenschaften wie die relative Ausrichtung ihrer optischen Achsen sowie die relative Anordnung zueinander im Raum, sowie ihre intrinsischen Eigenschaften, also die Eigenschaften der Kameras selbst, die definieren, wie ein Punkt im Raum, der sich im internen Koordinatensystem der jeweiligen Kamera befindet, auf die Koordinaten der Pixel des aufgenommenen Bildes abgebildet wird.

Der Kameraträger 14 umschließt den Innenraum 22 nur nach vorne, zur Säule 12 hin, und zu den Seiten, also links und rechts des Kopfes des Probanden. Nach oben, unten sowie zu einer Rückseite 30 hin ist er offen, wobei die freien Enden 18 zueinander einen Abstand von mindestens 25cm aufweisen, so dass sich der Proband bequem von der Rückseite aus nähern kann. Im gezeigten Ausführungsbeispiel beträgt der Abstand 70 bis 80cm.

Zur Ausleuchtung des Innenraums 22 ist eine Beleuchtungseinrichtung mit einer oberhalb der Kamerareihe 26 verlaufenden oberen Lichtleiste 32 sowie einer unterhalb der Kamerareihe 26 verlaufenden unteren Lichtleiste 34 vorgesehen, welche jeweils eine Vielzahl von LEDs als Leuchtmittel aufweisen. Die obere Lichtleiste 32 und die untere Lichtleiste 34 erstrecken sich jeweils durchgehend oder mit Unterbrechungen über eine Länge, die mindestens so groß ist wie die Länge der in Umfangsrichtung zwischen den freien Enden 18 gemessenen Länge der Kamerareihe 26. Diese entspricht einem Umfangswinkel von mindestens 160 Grad. Nahe den freien Enden 18 sind die obere Lichtleiste 32 und die untere Lichtleiste 34 jeweils mittels einer vertikal verlaufenden weiteren Lichtleiste 36 miteinander verbunden. Die Kamerareihe 26 wird somit vollständig durch mindestens eine Reihe LEDs umrahmt. Die Vorrichtung 10 weist zudem eine in der Zeichnung nicht näher dargestellte Steuer- oder Regeleinrichtung auf, mit der die von den LEDs abgestrahlte Lichtintensität abhängig von der durch die Kameras 16a, 16b detektierten Lichtintensität gesteuert oder geregelt werden kann. Die LEDs der Lichtleisten 32, 34, 36 sind dabei zu Sektoren zusammengefasst, deren abgestrahlte Lichtintensitäten getrennt voneinander gesteuert bzw. geregelt werden können. Zudem ist vorgesehen, dass auch die von den einzelnen LEDs abgestrahlten Lichtintensitäten mittels der Steuer- oder Regeleinrichtung getrennt voneinander gesteuert oder geregelt werden können.

Um den Probanden richtig im Innenraum 22 positionieren zu können, sind die beiden der Frontkamera 16a nächstgelegenen Seitenkameras 16b dazu eingerichtet, den Abstand des Kopfs des Probanden von der Mitte 38 des Kameraträgers 14 zu messen. Mittels einer nicht näher dargestellten Anzeigeeinheit wird dem Probanden angezeigt, ob er richtig steht oder nicht. Die Anzeigeeinheit weist mehrere unterschiedlich eingefärbte Lichtquellen auf, die in einer Reihe angeordnet sind. Die mittlere Lichtquelle leuchtet grün, wenn der Proband richtig steht. Ausgehend von der mittleren Lichtquelle gibt es in jeder Richtung in dieser Reihenfolge eine gelbe, eine orangene und eine rote Lichtquelle, die entsprechend der Farbe anzeigt, wenn der Proband ein wenig, deutlich oder viel zu weit von der Mitte 38 des Kameraträgers 14 entfernt bzw. ein wenig, deutlich oder viel zu nah zur Mitte 38 steht. Um bei der Bestimmung der Zentrierparameter sicherzustellen, dass die Blickrichtung des Probanden ins Unendliche gerichtet ist, ist eine am Kameraträger 14 angeordnete Fixationseinrichtung 42 vorgesehen, die ein Fixationsmuster für den Probanden in Form eines Specklemusters erzeugt. Das Fixationsmuster ist etwas höher angeordnet als die Frontkamera 16a, so dass der Proband über diese hinwegblickt. Damit kann sein Gesicht im größtmöglichen Umfang aufgenommen werden.

Die Vorrichtung 10 eignet sich insbesondere auch zur Herstellung eines Avatars des Kopfs des Probanden, welcher zur Bestimmung der Zentrierparameter herangezogen werden kann. Zum diesem Zweck werden durch die Kameras 16a, 16b kalibrierte Bilder des Kopfs des Probanden ohne Brille bzw. Brillenfassung aufgenommen. Mittels eines geeigneten Prozesses zur geometrischen Positionsbestimmung wie beispielsweise Triangulation wird ein Tiefenprofil des Kopfes erstellt, das diesen näherungsweise sehr gut abbildet. Der Kopf wird durch eine Vielzahl von Punkten abgebildet, die mittels eines Netzmusters miteinander verbunden werden können oder aber als Punktwolke gespeichert werden können. Bei der anschließenden Bestimmung der Zentrierparameter kann der so ermittelte Avatar herangezogen werden, um Zentrierparameter zu bestimmen, die aufgrund der geometrischen Eigenschaften der Brille bzw. Brillenfassung, die der Proband trägt, nicht oder nur näherungsweise bestimmt werden können. Beispielsweise kann ein breiter Fassungsbügel das Auge in einer Seitenaufnahme soweit verdecken, dass der Hornhautscheitelabstand nicht oder nur sehr ungenau bestimmt werden kann. Zudem können gefärbte oder stark spiegelnde Gläser die Augen nicht oder nur sehr schlecht erkennen lassen. Um dem zu begegnen, wird auf die von den Kameras 16a, 16b aufgenommenen Bilder des die Brille oder Brillenfassung tragenden Probanden das Tiefenprofil des Avatars projiziert und die Zentrierparameter, die aufgrund der durch die Brille bzw. Brillenfassung eingeschränkten Sicht nur ungenügend bestimmt werden können, werden mittels der Bilddaten des Avatars bestimmt. Dabei kann eine Anpassung des Avatars auf die Bilder des die Brille bzw. Brillenfassung tragenden Probanden zur Minimierung von Abweichungen erfolgen.

Die oben beschriebene Vorrichtung 10 kann wie folgt für eine pupillenbasierte Detektion eines Hornhautscheitels ebenso wie für eine reflexbasierte Detektion eines Hornhautscheitels bei beiden Augen des Probanden eingesetzt werden.

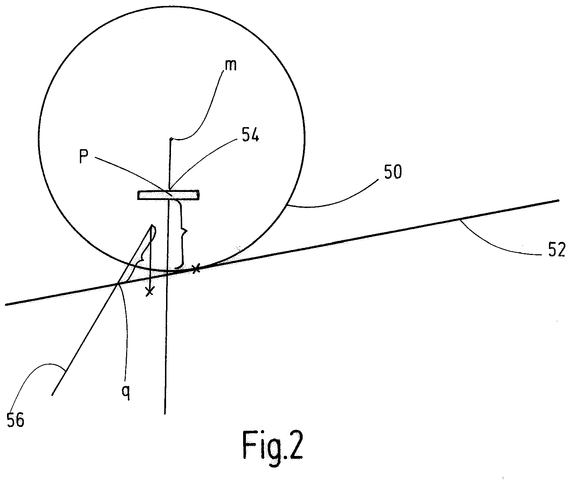

Bei der pupillenbasierten Methode gemäß Figur 2 wird zunächst die Position des Hornhautscheitels im Raum in erster Näherung als Schnittpunkt q eines die Hornhaut 50 tangierenden ersten Sichtstrahls 52 einer der ein Seitenbild des Probanden aufnehmenden Seitenkameras 16b mit einem auf die Pupille 54 gerichteten zweiten Sichtstrahl 56 der ein Frontbild des Probanden aufnehmenden Frontkamera 16a bestimmt. Mittels einer Korrekturrechnung wird eine korrigierte Position des Hornhautscheitels im Raum mittels der Gleichung a = q + µ ^{∗} v + µ ^{∗} w berechnet. Dabei ist µ ein Erfahrungswert oder ein tatsächlicher Wert für den Abstand zwischen dem Pupillenzentrum und dem Hornhautscheitel, der regelmäßig Werte zwischen 2,5mm und 4mm annimmt. v ist ein Einheitsvektor der Raumrichtung vom Pupillenzentrum p zur Frontkamera 16a, deren Koordinaten mit der Variable c1 angegeben werden, und errechnet sich als v = (p-c1) / | p - c1 |. w ist ein Einheitsvektor der durch das Zentrum m der Hornhautkugel verlaufenden Blickrichtung, die auf das Fixationsmuster der Fixationseinrichtung 42 am Raumpunkt t gerichtet ist, und errechnet sich zu w = (t - m) / | t - m |. Alle Werte a, q, p, c1, t und m sind dreidimensionale Vektoren.

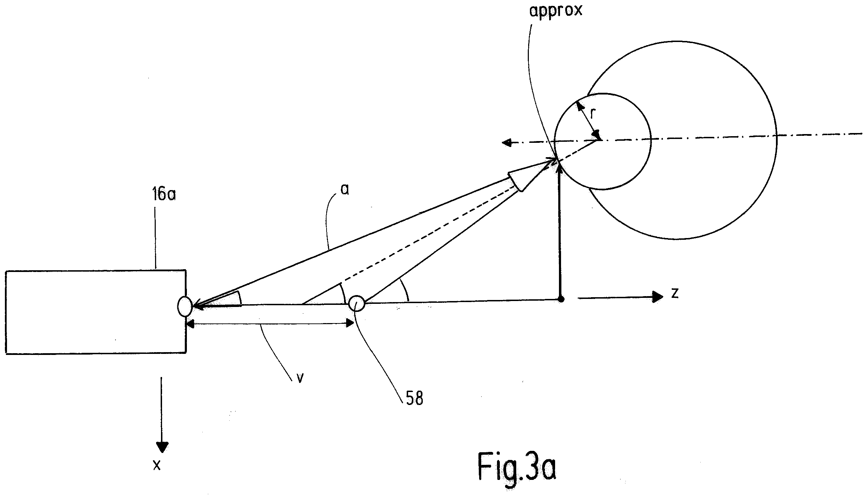

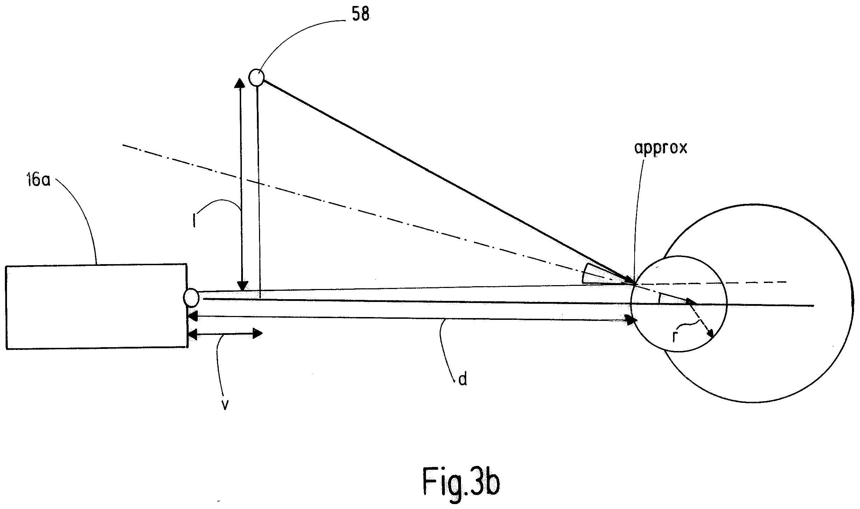

Bei der reflexbasierten Bestimmung der Position des Hornhautscheitels gemäß Figur 3a, 3b sind zwei Korrekturrechnungen vorzunehmen, wobei die erste Korrekturrechnung (Figur 3a) eine Korrektur in x-Richtung, die zweite Korrektur (Figur 3b) eine Korrektur in y-Richtung betrifft. Diese Raumrichtungen werden durch ein inneres Koordinatensystem der Frontkamera 16a festgelegt, das seinen Nullpunkt im optischen Zentrum der Frontkamera 16a hat. Die z-Richtung wird dabei durch die Blickrichtung der Frontkamera 16a festgelegt, die x-Richtung ist eine Richtung, die horizontal sowie orthogonal zur z-Richtung verläuft und in deren Richtung betrachtet nach rechts weist, und die y-Richtung verläuft orthogonal zur x-Richtung und zur z-Richtung und weist im Raum nach oben. Bei der reflexbasierten Messung wird mittels einer Lichtquelle, im vorliegenden Fall einer LED 58 ein Lichtblitz ausgesandt, dessen Reflexion auf der Hornhaut von der Frontkamera 16a und mindestens einer der Seitenkameras 16b detektiert wird und die erste Näherung für die Position des Hornhautscheitels im Raum bildet. Der Reflexpunkt wird in Figur 3a, 3b mit "approx" bezeichnet. In x-Richtung wird eine Korrektur durch Addition von Δx =+/ -r ^{∗} sin (½ ^{∗} (arccos z/a + arctan x/(z-v))) zur x-Koordinate des Reflexpunkts approx vorgenommen, wobei das Pluszeichen bei der Anwendung auf das linke Auge, das Minuszeichen bei der Anwendung auf das rechte Auge (vgl. Figur 3a) zu verwenden ist. r ist dabei ein Erfahrungswert oder ein tatsächlicher Wert für den Hornhautradius, der typischerweise ca. 8mm beträgt. a ist der Abstand des optischen Zentrums der Frontkamera 16a zum Reflexpunkt approx und v ist der Abstand der LED 58 zum optischen Zentrum der Frontkamera 16a in z-Richtung. x und z wiederum sind die Koordinaten in x- und z-Richtung.

In y-Richtung wird ausgehend vom Reflexpunkt approx eine Korrektur mittels Addition von Δy =+/ -r ^{∗} sin ½ ^{∗} (arctan I/(d-v)) vorgenommen. r ist wiederum der Erfahrungswert oder ein tatsächlicher Wert für den Hornhautradius, d ist der Abstand des optischen Zentrums der Frontkamera 16a zum Reflexpunkt approx in z-Richtung, v ist der Abstand der LED 58 zum optischen Zentrum der Frontkamera 16a in z-Richtung und I ist der Abstand der LED 58 vom optischen Zentrum der Frontkamera 16a in y-Richtung. Das Pluszeichen kommt zur Anwendung, wenn die LED 58 unter der Frontkamera 16a angeordnet ist, also ihre y-Koordinate kleiner ist als die y-Koordinate der Frontkamera 16a bzw. ihres optischen Zentrums. Ist die LED über der Frontkamera 16a angeordnet, so kommt das Minuszeichen zum Einsatz.

Bei den beschriebenen Verfahren kann die Pupille bzw. der Reflexpunkt approx beispielsweise mittels Merkmalsextraktion, mittels Merkmalsmatching und/oder mittels maschinellem Lernen durch Vergleich mit einer Vielzahl vorbekannter Daten detektiert werden. Diesem Detektionsschritt kann ein Schritt vorangehen, bei dem ein Gesichtsdetektor erkennt, welche Bildpunkte zum Gesicht des Probanden bzw. zu seiner Augenpartie gehören, so dass nach der Pupille bzw. dem Reflexpunkt approx bereits eingegrenzt gesucht werden kann.

## Patentansprüche

1. Computerimplementiertes Verfahren zur Detektion eines Hornhautscheitels, wobei ein Frontbild eines Kopfes und ein Seitenbild des Kopfes bereitgestellt werden, die zueinander kalibriert sind, wobei aus dem Frontbild und dem Seitenbild mittels geometrischer Positionsbestimmung die Position des Hornhautscheitels im Raum bestimmt wird, **dadurch gekennzeichnet, dass** die Position des Hornhautscheitels im Raum in einer ersten Näherung als Schnittpunkt q eines die Hornhaut (50) tangierenden ersten Sichtstrahls (52) einer das Seitenbild aufnehmenden Seitenkamera (16b) mit einem auf die Pupille (54) gerichteten zweiten Sichtstrahl (56) einer das Frontbild aufnehmenden Frontkamera (16a) bestimmt wird und dass die Pupille mittels Merkmalsextraktion und/oder Merkmalsmatchings und/oder mittels Maschinellen Lernens durch Vergleich mit einer Vielzahl vorbekannter Daten detektiert wird.

2. Computerimplementiertes Verfahren nach Anspruch 1, **dadurch gekennzeichnet, dass** aus dem Frontbild ein auf den Hornhautscheitel gerichteter Sichtstrahl der Frontkamera (16a) berechnet und in das Seitenbild projiziert wird und dass der Hornhautscheitel auf dem ins Seitenbild projizierten Sichtstrahl detektiert wird.

3. Computerimplementiertes Verfahren nach einem der vorangehenden Ansprüche, **dadurch gekennzeichnet, dass** die Aufnahmerichtung des Frontbilds mit der Aufnahmerichtung des Seitenbilds und gegebenenfalls mit der Aufnahmerichtung des weiteren Seitenbilds einen Winkel von mehr als 60 bis 120 Grad und vorzugsweise von 90 Grad einschließt.

4. Vorrichtung zur Detektion eines Hornhautscheitels mit einer Frontkamera (16a) zur Aufnahme eines Frontbilds, mit einer Seitenkamera (16b) zur Aufnahme eines Seitenbilds und mit einem Computer, welcher einen Speicher aufweist, in dem ein Computerprogramm mit Programmcode zur Durchführung aller Verfahrensschritte nach einem der Ansprüche 1 oder 2 gespeichert ist, und welcher einen Prozessor aufweist, der eingerichtet ist, um das im Speicher abgespeicherte Computerprogramm auszuführen.

5. Verfahren zum Zentrieren mindestens eines Brillenglases in einer Brillenfassung, **dadurch gekennzeichnet, dass** in einem ersten Verfahrensschritt die Position mindestens eines Hornhautscheitels im Raum mit einem Verfahren nach einem der Ansprüche 1 bis 3 detektiert wird, dass in einem zweiten Verfahrensschritt unter Heranziehung der Position des mindestens einen Hornhautscheitels im Raum mindestens ein Zentrierparameter bestimmt wird und dass in einem dritten Verfahrensschritt das mindestens eine Brillenglas mit dem bzw. den im zweiten Verfahrensschritt bestimmten Zentrierparameter(n) in der Brillenfassung zentriert wird.

6. Verfahren zum Einschleifen mindestens eines Brillenglases in eine Brillenfassung, **dadurch gekennzeichnet, dass** in einem ersten Verfahrensschritt die Position mindestens eines Hornhautscheitels im Raum mit einem Verfahren nach einem der Ansprüche 1 bis 3 detektiert wird, dass in einem zweiten Verfahrensschritt unter Heranziehung der Position des mindestens einen Hornhautscheitels im Raum mindestens ein Zentrierparameter bestimmt wird und dass in einem dritten Verfahrensschritt das mindestens eine Brillenglas basierend auf dem bzw. den im zweiten Verfahrensschritt bestimmten Zentrierparameter(n) für eine Anordnung in der Brillenfassung eingeschliffen wird.

7. Verfahren zum Herstellen eines Brillenglases, **gekennzeichnet durch** die Verfahrensschritte gemäß Anspruch 6.

8. Verfahren zum Herstellen einer Brille, **dadurch gekennzeichnet, dass** ein Verfahren nach einem der Ansprüche 5 bis 7 verwendet wird.

## Claims

1. Computer-implemented method for detecting a cornea vertex, wherein a frontal image of a head and a lateral image of the head are provided, said images being calibrated to one another, wherein the position of the cornea vertex in space is determined from the frontal image and the lateral image by means of a geometric position determination, **characterized in that** the position of the cornea vertex in space is determined to a first approximation as the point of intersection q of a first view ray (52), tangential to the cornea (50), from a lateral camera (16b) recording the lateral image with a second view ray (56), directed onto the pupil (54), from a frontal camera (16a) recording the frontal image and **in that** the pupil is detected by means of feature extraction and/or feature matching and/or by means of machine learning by comparison with a multiplicity of data known in advance.

2. Computer-implemented method according to Claim 1, **characterized in that** a view ray of the frontal camera (16a) directed to the cornea vertex is calculated from the frontal image and said view ray is projected into the lateral image, and **in that** the cornea vertex is detected on the view ray that is projected into the lateral image.

3. Computer-implemented method according to either of the preceding claims, **characterized in that** the recording direction of the frontal image forms an angle of more than 60 to 120 degrees and preferably of 90 degrees with the recording direction of the lateral image and if necessary with the recording direction of the further lateral image.

4. Apparatus for detecting a cornea vertex, having a frontal camera (16a) for recording a frontal image, having a lateral camera (16b) for recording a lateral image and having a computer, which comprises a memory, in which a computer program having program code for implementing all of the method steps according to either of Claims 1 and 2 is stored, and which comprises a processor which is configured to execute the computer program stored in the memory.

5. Method for centring at least one spectacle lens in a spectacle frame, **characterized in that** in a first method step the position of at least one cornea vertex in space is detected with a method according to any of Claims 1 to 3, **in that** in a second method step at least one centring parameter is determined using the position of the at least one cornea vertex in space and **in that** in a third method step the at least one spectacle lens is centred in the spectacle frame using the centring parameter or parameters determined in the second method step.

6. Method for fitting at least one spectacle lens in a spectacle frame by grinding, **characterized in that** in a first method step the position of at least one cornea vertex in space is detected with a method according to any of Claims 1 to 3, **in that** in a second method step at least one centring parameter is determined using the position of the at least one cornea vertex in space and **in that** in a third method step the at least one spectacle lens is ground on the basis of the centring parameter or parameters determined in the second method step for an arrangement in the spectacle frame.

7. Method for producing a spectacle lens, **characterized by** the method steps according to Claim 6.

8. Method for producing a pair of spectacles, **characterized in that** a method according to any of Claims 5 to 7 is used.

## Revendications

1. Procédé mis en œuvre par ordinateur pour détecter un apex cornéen, une image frontale d'une tête et une image latérale de la tête étant fournies, lesquelles sont étalonnées l'une par rapport à l'autre, la position de l'apex cornéen dans l'espace étant déterminée à partir de l'image frontale et de l'image latérale par détermination de la position géométrique, **caractérisé en ce que** la position de l'apex cornéen dans l'espace est déterminée en première approximation comme l'intersection q d'un premier faisceau de visée (52), tangent à la cornée (50), d'une caméra latérale (16b) enregistrant l'image latérale avec un deuxième faisceau de visée (56), dirigé vers la pupille (54), d'une caméra frontale (16a) enregistrant l'image frontale et **en ce que** la pupille est détectée par comparaison avec un grand nombre de données précédemment connues par extraction de caractéristiques et/ou correspondance de caractéristiques et/ou apprentissage automatique.

2. Procédé mis en œuvre par ordinateur selon la revendication 1, **caractérisé en ce qu'**un faisceau de visée, dirigé vers l'apex cornéen de la caméra frontale (16a), est calculé et projeté dans l'image latérale à partir de l'image frontale et **en ce que** l'apex cornéen est détecté sur le faisceau de visée projeté dans l'image latérale.

3. Procédé mis en œuvre par ordinateur selon l'une des revendications précédentes, **caractérisé en ce que** la direction d'enregistrement de l'image frontale forme un angle supérieur à 60-120 degrés et de préférence 90 degrés avec la direction d'enregistrement de l'image latérale et éventuellement avec la direction d'enregistrement de l'autre image latérale.

4. Dispositif de détection d'un apex cornéen, ledit dispositif comprenant une caméra frontale (16a) destinée à enregistrer une image frontale, une caméra latérale (16b) destinée à enregistrer une image latérale et un ordinateur qui comporte une mémoire dans laquelle est mémorisé un programme informatique muni d'un code de programme pour effectuer toutes les étapes du procédé selon l'une des revendications 1 et 2, et qui comporte un processeur qui est configuré pour exécuter le programme informatique mémorisé dans la mémoire.

5. Procédé de centrage d'au moins un verre de lunettes dans une monture de lunettes, **caractérisé en ce que** la position d'au moins un apex cornéen dans l'espace est détectée dans une première étape de procédé à l'aide d'un procédé selon l'une des revendications 1 à 3, **en ce que**, dans une deuxième étape de procédé, au moins un paramètre de centrage est déterminé dans l'espace à l'aide de la position de l'au moins un apex cornéen et **en ce que**, dans une troisième étape de procédé, l'au moins un verre de lunettes est centré dans la monture de lunettes avec l'au moins un paramètre de centrage déterminé dans la deuxième étape de procédé.

6. Procédé d'insertion par meulage d'au moins un verre de lunettes dans une monture de lunettes, **caractérisé en ce que,** dans une première étape de procédé, la position d'au moins un apex cornéen dans l'espace est détectée à l'aide d'un procédé selon l'une des revendications 1 à 3, **en ce qu'**au moins un paramètre de centrage est déterminé dans une deuxième étape de procédé à l'aide de la position de l'au moins un apex cornéen dans l'espace et **en ce que**, dans une troisième étape de procédé, l'au moins un verre de lunettes est inséré par meulage dans la monture de lunettes sur la base de l'au moins un paramètre de centrage déterminé dans la deuxième étape de procédé.

7. Procédé de fabrication d'un verre de lunettes, **caractérisé par** les étapes de procédé selon la revendication 6.

8. Procédé de fabrication d'un verre de lunettes, **caractérisé en ce que** le procédé selon l'une des revendications 5 à 7 est utilisé.
